Europäisches Patentamt

European Patent Office

Office européen des brevets

(19)

(11) Numéro de publication: **0 129 531 B1**

(12) **FASCICULE DE BREVET EUROPEEN**

(45) Date de publication de fascicule du brevet:
02.05.91

(51) Int. Cl.⁵: **A61F 2/30**

(21) Numéro de dépôt: **84870080.3**

(22) Date de dépôt: **15.06.84**

(54) **Procédé et moyen pour la réalisation d'une prothèse.**

(30) Priorité: **17.06.83 NL 8302178**

(43) Date de publication de la demande:
**27.12.84 Bulletin 84/52**

(45) Mention de la délivrance du brevet:
**02.05.91 Bulletin 91/18**

(84) Etats contractants désignés:
**AT BE CH DE FR GB IT LI LU NL SE**

(56) Documents cités:
**EP-A- 0 006 415      EP-A- 0 035 481**
**EP-A- 0 054 785      EP-A- 0 061 993**
**FR-A- 1 456 600      FR-A- 2 262 955**
**US-A- 4 051 598      US-A- 4 253 829**

(73) Titulaire: **N.V. STEWAL S.A.**
**rue Ulens, 10**
**B-1020 Bruxelles(BE)**

(72) Inventeur: **Mulier, Joseph Cyriel**
**Waversebaan 13**
**B-3031 Oud Heverlee(BE)**

(74) Mandataire: **Claeys, Pierre et al**
**Bureau Gevers rue de Livourne 7 Bte 1**
**B-1050 Bruxelles(BE)**

## Description

L'invention concerne un procédé de fabrication d'un élément de prothèse, tel que décrit dans le préambule de la revendication 1.

Les prothèses implantables sont généralement connues et souvent employées pour le remplacement en entier ou en partie d'une articulation de hanche, genou ou épaule.

A cet effet l'ancrage doit être conçu de façon à ne pas déchirer les parties restantes du squelette et de façon à ce qu'une suture résistante soit assurée.

Pour l'ancrage d'une prothèse dans une partie de squelette, il existe diverses méthodes. Suivant une de ces méthodes, la tige de la prothèse est enfoncée avec force dans la cavité médullaire et il en résulte une suture résistante forcée. Les inconvénients de cette méthode sont, d'une part, que l'os peut casser ou se déchirer lors de l'enfoncement de la tige et, d'autre part, qu'à la longue la suture peut s'user et qu'ainsi la prothèse se détache.

Suivant d'autres méthodes, la tige de la prothèse est glissée dans une cavité préparée à l'avance dans le segment d'os, pour que la cavité soit suffisamment vaste pour rendre ce glissement facile. Ensuite on fixe la prothèse à l'os soit par des moyens mécaniques tels que des vis, soit à l'aide de moyens chimiques, tels qu'un ciment à durcissement rapide à base de polyméthylméthacrylate ou encore à l'aide d'une masse poreuse qui accélère le développement naturel des tissus osseux.

Ces méthodes, dont on s'est satisfait jusqu'à présent présentent tout de même des inconvénients. On constate en effet que lorsque la prothèse est fixée avec des vis, ces dernières se dévissent après un certain temps, ce qui provoque du jeu dans la prothèse. Dans le cas de l'apport d'une masse poreuse à la surface de la prothèse, on est confronté à une méthode très fastidieuse. Tandis que, avec l'emploi d'un ciment, il est difficile de centrer la prothèse dans la cavité. De plus, il peut se produire une interruption de la couche de ciment à certains endroits, ce qui a pour résultat de provoquer une transmission irrégulière de la charge mécanique de la prothèse vers l'os.

Il est évident, vu ces inconvénients, qu'il était intéressant d'améliorer les méthodes d'ancrage d'une prothèse sur une partie de squelette.

Au cours d'essais, qui ont mené à l'invention, il est apparu qu'une bonne suture de la prothèse à un segment d'os pouvait également s'obtenir, sans employer les méthodes mécaniques, chimiques ou faisant appel à une masse poreuse précitées, si, dans une cavité préformée d'un segment d'os, on glisse la tige de la prothèse, qui est faite sur mesure, pour aboutir, sans avoir recours à la force et à des chocs, à un emboîtement ajusté et à un placement régulier de la prothèse contre l'os, ce qui répartirait les tensions dans l'os et favoriserait son développement naturel.

Dans ce sens, on connait un procédé du type cité au début (voir EP-A-054725). Ce procédé reste toutefois encore insatisfaisant quant à sa précision.

L'invention a pour but, en vue d'éviter les inconvénients des méthodes connues d'ancrage de prothèses, de procurer un procédé suivant lequel la prothèse ou au moins la partie d'ancrage de celle-ci, peut être fabriquée de telle sorte que cette partie, lors de son introduction par glissement dans la cavité préformée dans l'os ou une autre partie de squelette, peut s'ajuster de manière précise.

On résout ce problème, suivant l'invention, par un procédé tel que cité au début et présentant les particularités caractérisantes de la revendication 1 Des modes de réalisation avantageux et particuliers de l'invention ressortent en outre des revendications secondaires.

De cette manière on peut, dans un temps relativement court, fabriquer une prothèse sur mesure, qui peut alors être glissée dans la cavité formée, pour l'obtention d'un ancrage par ajustage précis.

La formation d'une cavité dans une partie de squelette est connue en soi et peut se faire de manière courante, par exemple par l'évacuation de la cavité médullaire.

Suivant l'invention, on procède avantageusement, pour la réalisation de l'empreinte, de la manière suivante : on remplit la cavité formée à l'aide d'une matière stérile capable d'épouser les parois de ladite cavité, cette matière étant choisie parmi les matières non adhérentes, durcissant à froid et possédant, à l'état durci, un module d'élasticité tel que la matière durcie puisse être extraite de la cavité sans déformation permanente, on laisse durcir la matière dans la cavité et on l'enlève ensuite de cette dernière. Cette façon de procéder est simple, exige peu de temps et permet d'obtenir une empreinte maniable, dont les dimensions peuvent être relevées de manière précise et qui peut ultérieurement être travaillée.

Suivant l'invention, pour copier l'empreinte afin de fabriquer au moins la partie de prothèse à fixer dans la cavité, on relève les contours de l'empreinte pour en déterminer les côtes et on transmet les données relevées à des moyens de façonnage, pour conduire ces derniers, afin de réaliser la partie de prothèse précitée à partir d'une ébauche grossière en un matériau convenant pour prothèse.

Ces opérations peuvent se faire en peu de temps, de façon à obtenir rapidement la prothèse ou la partie de prothèse désirée à fixer dans la cavité susdite.

Pour la réalisation de l'empreinte décrite ci-dessus, des moyens très simples sont utilisés, ceux-ci comprenant au moins une matière stérile non adhérente, du type à deux composants, durcissant à froid et possédant, à l'état durci, un module d'élasticité tel qu'elle puisse être dégagée de la cavité et des moyens pour injecter cette matière dans ladite cavité.

Pour copier les empreintes, le dispositif nécessaire comprend des moyens pour relever les contours de l'empreinte, des moyens de fabrication de la prothèse à partir d'une ébauche et des moyens pour transmettre, après corrections éventuelles, les données relevées aux moyens de fabrication pour conduire cette dernière.

D'autres détails et particularités de l'invention ressortiront de la description des dessins annexés au présent mémoire et qui illustrent plusieurs formes d'exécution du procédé, des moyens et du dispositif susdits, à titre d'exemples non limitatifs plus spécialement axés sur la réalisation d'une partie d'une prothèse de la hanche.

La figure 1 représente schématiquement un premier mode d'exécution d'une empreinte de la cavité réalisée dans un segment d'os.

Les figures 2 et 3 représentent schématiquement une seconde forme d'exécution d'une telle empreinte, à deux stades successifs.

La figure 4 représente schématiquement le sachet utilisé dans le cadre des formes d'exécution selon les figures 1 à 3.

La figure 5 montre schématiquement une troisième forme d'exécution d'une empreinte.

La figure 6 est une vue en coupe, suivant la ligne VI-VI de la figure 5.

La figure 7 montre, à titre de produit intermédiaire, une empreinte obtenue selon la forme d'exécution des figures 2 et 3.

Les figures 8 et 9 montrent une forme d'exécution de la manière dont est copié ce produit intermédiaire en vue d'obtenir la prothèse souhaitée, et cela à deux stades successifs.

La figure 10 montre une variante de la prothèse représentée en cours de fabrication à la figure 7, cette variante étant réalisée en deux pièces, celles-ci étant représentées non assemblées.

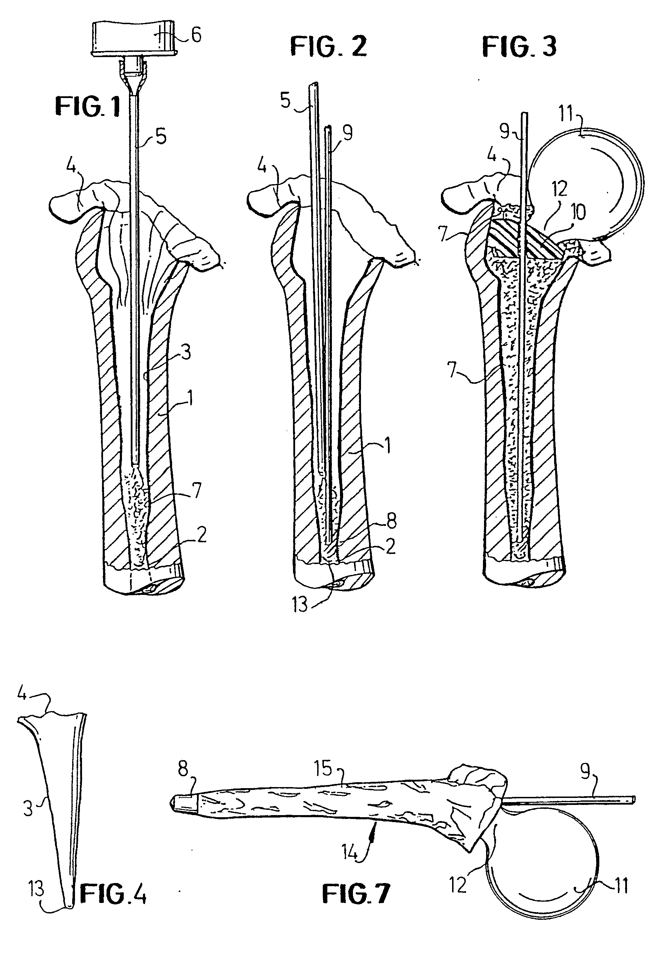

La figure 1 illustre un segment d'un fémur 1 faisant partie du corps humain ou appartenant à un animal et dans lequel une partie d'une prothèse de la hanche doit être fixée. On forme tout d'abord dans ce fémur une cavité 2 par évacuation de la cavité médullaire. On dispose ensuite dans la cavité 2 un sachet 3 en un matériau en feuille non adhérent et cela de telle sorte que la partie supérieure ouverte 4 de celui-ci dépasse du fémur 1. Dans le sachet est alors placé un conduit d'injection 5 qui est raccordé à un cylindre 6 rempli d'une matière durcissable. Tous ces éléments et moyens ont été stérilisés au préalable. La matière qui se trouve dans le cylindre 6 est injectée via le conduit 5 dans le sachet 3 à l'aide de moyens de pression non représentés, tels qu'un pistolet d'injection. A mesure que le niveau de la matière 7 monte dans le sachet, on dégage progressivement le conduit d'injection 5 et le cylindre 6 jusqu'à ce que le sachet soit entièrement rempli de matière. On évacue ensuite le conduit 5 et le cylindre 6 et on laisse durcir "in situ" la matière 7, ce qui prend environ 4-5 minutes. Après durcissement de la matière, le sachet 3 et son contenu peuvent aisément être retirés de la cavité 2 de l'os et on est en possession d'une empreinte précise de cette dernière.

La forme d'exécution de l'empreinte illustrée aux figures 2 et 3 est basée sur le même principe que celle montrée à la figure 1. On dispose au fond du sachet 3, placé dans la cavité 2 un bouchon 8 en un matériau caoutchouteux élastique, dans lequel est fixée une tige de centrage métallique 9 qui s'étend sur toute la hauteur du sachet jusqu'au-delà de la partie supérieure ouverte 4 de celui-ci. Le conduit d'injection 5 est alors introduit dans le sachet pour le remplir de matière 7.

Après le retrait du conduit d'injection 5, lorsque le sachet est rempli de matière, on fixe sur la tige de centrage 9 un bouchon 10 qui comprime ladite matière et la repousse partiellement latéralement. Sur ce bouchon 10 est fixée une reproduction de tête de prothèse 11 présentant une partie en forme de col 12 et le bouchon 10 est réalisé de telle sorte que la reproduction 11 est dirigée dans le bon sens. Après durcissement de la matière, le sac est enlevé sans peine de la cavité 2 et on a obtenu une empreinte de celle-ci.

A la forme d'exécution selon les figures 2 et 3, diverses variantes possibles peuvent être apportées. C'est ainsi que le conduit d'injection 5 peut être disposé coaxialement autour de la tige de centrage 9. Le bouchon 10 pourrait être disposé à l'extrémité supérieure du sachet 3 avant l'introduction de la matière, le conduit d'injection 5 étant alors glissé par un orifice ménagé dans le bouchon 10 pour remplir le sac 3 de matière 7.

La figure 4 représente une forme de réalisation du sachet 3 pouvant être utilisée dans le cadre des prises d'empreinte selon les figures 1 à 3. Ce sachet présente la forme d'un cône à partir de sa partie supérieure ouverte 4 vers son extrémité inférieure obturée 13. La figure 7 montre l'empreinte de la cavité 2 obtenue par le procédé illustré aux figures 2 et 3, c'est-à-dire un produit intermédiaire 14 à partir duquel la prothèse sera fabriquée. Le sachet 3 a été enlevé et le produit intermédiaire 14 consiste essentiellement en un corps ou un manchon 15 constitué de la matière 7 durcie, qui présente à une de ses extrémités le bouchon 8 en un matériau caoutchouteux élastique et à l'autre extrémité la reproduction de la tête de prothèse 11 présentant le col 12. La tige de centrage 9, qui traverse le corps 15 à partir du bouchon de caoutchouc 8, fait saillie à l'extérieur à partir de l'extrémité opposée au bouchon 8 et peut alors faire office, avec ce dernier, d'axe de pivotement pour ce produit intermédiaire.

Dans la forme de réalisation de l'empreinte illustrée aux figures 5 et 6, on dispose, dans la cavité 2, la tige de centrage 9 pourvue, à son extrémité située dans la cavité, d'un bouchon 8. On injecte la matière 7 et pendant que celle-ci est encore à l'état pâteux, on enfonce dans cette matière une armature 38, qui est enfilée sur la tige de centrage 9. Cette armature 38 a une section qui décroit régulièrement de l'ouverture de la cavité 2 vers le fond de cette dernière, de sorte que, lorsqu'elle est enfoncée dans la matière, elle applique ladite matière régulièrement contre les parois de la cavité 2 pour qu'elle épouse étroitement toutes les irrégularités de l'entièreté de ces parois ainsi que les bords de l'ouverture de la cavité. Cette armature 38 présente avantageusement la reproduction de la tête de prothèse 11 qui peut être orientée, avant que la matière soit durcie, dans la direction voulue. Cette reproduction 11 peut être garnie d'un élément métallique 39 qui permet de constater, par radiographie, l'orientation de la reproduction de la tête de prothèse.

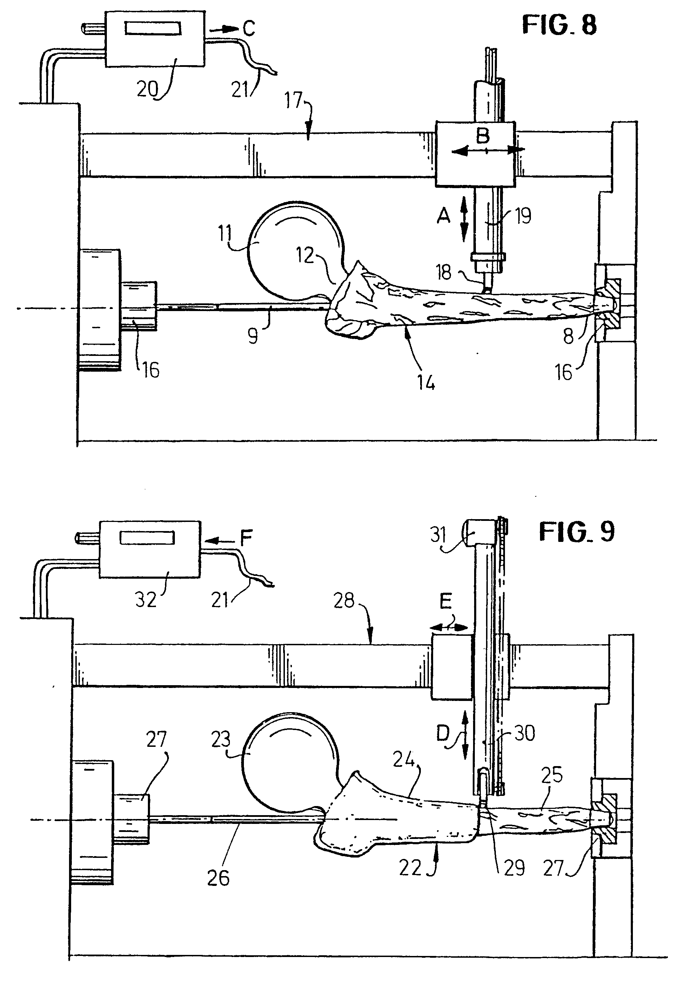

Les figures 8 et 9 représentent schématiquement la manière suivant laquelle la prothèse proprement dite peut être réalisée à partir d'un produit intermédiaire selon la figure 7.

Comme montré à la figure 8, le produit intermédiaire 14 est maintenu, par le bouchon 8 et la tige de centrage 9, de façon à pouvoir tourner dans des tourillons 16, d'un dispositif de prise de mesures 17. Un palpeur 18 est monté dans ce dispositif de façon à pouvoir se déplacer horizontalement et verticalement, suivant les flèches A et B, guidé dans le châssis du dispositif 17. Lorsqu'on fait tourner, autour de son axe, le produit intermédiaire 14, à l'aide des tourillons 16, le palpeur 18 suivra les contours de ce produit intermédiaire ou tout au moins du corps 15. Les données résultantes de cette opération sont alors converties en valeurs numériques dans un convertisseur 20 et fixées sur une bande perforée 21 qui quitte le convertisseur dans le sens de la flèche C.

La figure 9 montre le stade suivant du procédé, c'est-à-dire la formation de la prothèse proprement dite. Une ébauche 22 en matériau pour prothèse, présentant une tête 23 et un corps ou manchon 24, est montée à rotation à l'aide d'une tige de centrage 26 dans les tourillons 27 d'une machine de fabrication. Une meule ou fraise 29 est montée, grâce à un élément 30, de manière à pouvoir être déplacée aussi bien horizontalement que verticalement, suivant les flèches D et E, dans le châssis 28 de la machine, cette meule pouvant être mise en rotation grâce à un moteur 31. De plus, la machine comporte un convertisseur 32 grâce auquel les valeurs numériques de la bande perforée 21, introduite dans le sens de la flèche F, peuvent être converties en signaux de commande pour la meule 29. Lorsque l'ébauche 22 sera mise en rotation, à l'aide des tourillons 27, et que la bande perforée 21 défilera dans le convertisseur 32, la meule 29 effectuera un mouvement le long de la surface du corps 24 et façonnera celui-ci jusqu'à l'obtention d'un corps 25 ayant exactement les mêmes contours que le corps 15 du produit intermédiaire 14 dont les dimensions ont été relevées comme montré à la figure 8. On obtient de la sorte un élément de prothèse qui est une copie exacte du produit intermédiaire14. Il est évident que diverses variantes de la méthode en deux étapes suivant les figures 8 et 9 sont possibles. C'est ainsi qu'on peut éventuellement combiner en une seule installation les dispositif et machine selon les figures 8 et 9 en supprimant les convertisseurs 20, 32 et la bande perforée 21, les données relevées par le palpeur 18 étant transmises immédiatement à la meule 29 dans le but de commander celle-ci.

On peut également prévoir, suivant l'invention, préalablement à la transmission des données relevées par le palpeur 18 à la machine de fabrication de la prothèse, de traiter ces données pour éliminer les antidépouilles de l'empreinte 14, ce traitement des données s'effectuant en agissant sur la bande 21 pour en modifier les valeurs numériques suivant des critères prédéterminés.

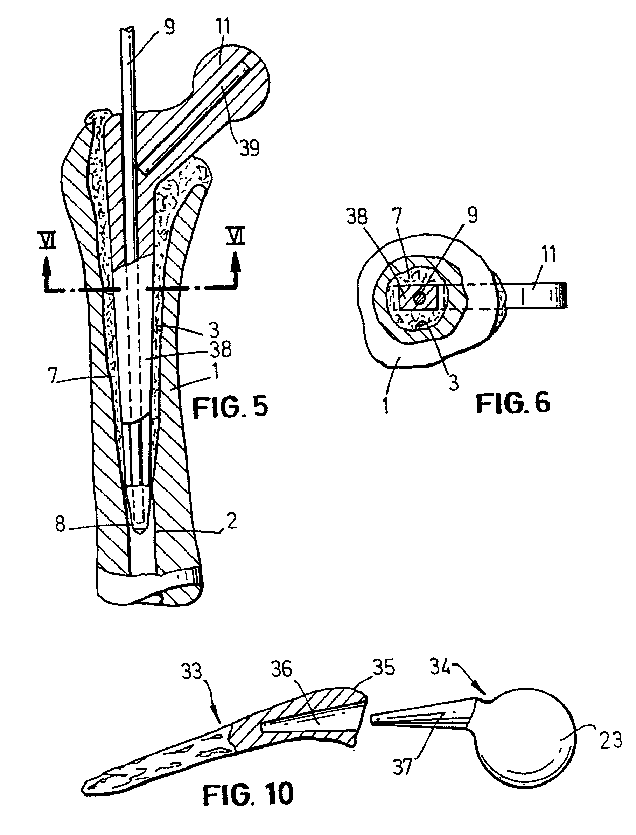

On peut aussi prévoir, suivant l'invention et comme montré à la figure 10, une prothèse réalisée en deux parties 33 et 34. La première partie 33, qui est façonnée, comme exposé ci-dessus, à partir de l'empreinte de la cavité 2 réalisée dans le fémur, est destinée à être logée dans cette cavité tandis que la seconde partie 34, qui présente la tête 23 de la prothèse, est préfabriquée. La partie 33 présente, suivant son axe et à partir de son extrémité 35, un évidement tronconique 36 destiné à recevoir un prolongement 37, de forme tronconique correspondante, du col de la tête de prothèse 23 préfabriquée. La rotation du prolongement 37 dans l'évidement 36 permet de corriger l'antéversion et la rétroversion.

Pour permettre de dégager la prothèse de la cavité, en cas de besoin, des moyens non représentés sont prévus sur la partie 33 de prothèse et agencés pour qu'on puisse y fixer un organe de traction pouvant agir suivant l'axe de cette partie 33 et permettant l'extraction de cette dernière de la cavité 2. Ces moyens peuvent par exemple être constitués par un filet réalisé dans l'évidement 36 et qui peut recevoir l'extrémité filetée correspondante de l'organe de traction.

Pour la mise en oeuvre du procédé complet suivant l'invention, on constate que l'on ne doit disposer, d'une part, que d'un nombre réduit de moyens auxiliaires simples en vue de produire une empreinte, moyens tels qu'un sachet (facultatif) constitué d'un matériau en feuille non adhérent, d'une matière première pouvant se mettre facilement en forme durcissant et à froid ainsi que des moyens pour introduire cette matière soit dans le sachet, soit directement dans la cavité 2 et, d'autre part, une installation de copiage de l'empreinte, telle que représentée aux figures 8 et 9.

Le matériau en feuille constituant le sachet 3 ne peut pas adhérer à la partie du squelette et, de préférence, il ne peut pas adhérer non plus à la matière destinée à constituer l'empreinte. De plus ce matériau doit pouvoir être stérilisé, par exemple à l'aide de vapeur. Comme matériau, le polyéthylène a donné de bons résultats, quoi que d'autres polymères thermoplastiques seraient également utilisables. Le cas échéant, on peut également faire usage d'une matière en feuille plus élastique que le polyéthylène pour la réalisation du sachet 3 dont la forme peut être cylindrique ou de préférence conique, comme montré à la figure 4.

La matière susdite doit, d'une part, pouvoir être coulée ou injectée et, d'autre part, durcir rapidement à la température ambiante tandis qu'elle doit également pouvoir être stérilisée, par exemple à l'aide de vapeur. Un élastomère siliconé à base de polydiméthylsiloxane, qui se présente sous la forme d'un système à deux composants avec un durcisseur (peroxyde + accélérateur habituel), a donné de bons résultats. Le durcisseur doit être ajouté peu avant l'emploi et être mélangé de manière homogène au polysiloxane afin de réaliser un durcissement régulier. La matière peut éventuellement être diluée à l'aide d'un polysiloxane liquide.

Après durcissement cette matière formera un corps compact ayant suffisamment de dureté pour résister à la pression du palpeur 18. Le sachet 3 qui entoure la matière durcie est enlevé, comme illustré à la figure 7, avant le copiage de l'empreinte. Mais, dans le cas où le sachet est constitué d'un matériau plus élastique que le polyéthylène, il pourra, sans problème, être conservé sur le produit intermédiaire.

Le bouchon 8 susdit est réalisé généralement en un matériau caoutchouteux élastique, tandis que le bouchon 10 et la reproduction de la tête de prothèse 11 ou l'armature 38 peuvent être réalisés en un matériau quelconque, la tige de centrage 9 étant métallique. Tous ces éléments doivent pouvoir être stérilisés.

Les opérations en vue de réaliser une empreinte peuvent être exécutées dans une salle d'opération, tandis que les opérations de copiage de l'empreinte peuvent être exécutées en un autre endroit. Vu la durée relativement courte de ces dernières opérations, il est également possible de faire exécuter les opérations de copiage dans un lieu proche de la salle d'opération.

## Revendications

1. Procédé de fabrication d'un élément de prothèse implantable, en matériau dur, pour le remplacement total ou partiel d'une articulation, en particulier d'une articulation de hanche, et destiné à épouser les formes d'une cavité (2) réalisée dans la partie du squelette (1) concernée, comprenant la prise d'une empreinte (14) de cette cavité, et la réalisation d'au moins une partie de l'élément de prothèse à partir de ladite empreinte, par transmission de données relatives à l'empreinte à un moyen d'enregistrement, traitement de ces données enregistrées et transmission des données traitées à des moyens de façonnage capables de réaliser, suivant les données transmises, l'élément de prothèse précité à partir d'une ébauche en un matériau dur convenant pour une prothèse, caractérisé en ce que, pour la prise d'une empreinte de la cavité, le procédé comprend un garnissage des parois de la cavité (2) par un sachet (3) réalisé en un matériau étanche et élastique, qui est stérile, souple et non adhérent auxdites parois, pour que son ouverture débouche à l'extérieur de la cavité, un remplissage du sachet (3) par une matière stérile (7), capable d'épouser les parois de la cavité, qui est choisie parmi les matières non adhérentes au matériau constituant le sachet, durcit à froid et possède, à l'état durci, un module d'élasticité tel que la matière durcie puisse être extraite de la cavité sans déformation permanente, et un enlèvement de la matière enfermée dans le sachet (3), après son durcissage dans la cavité, et en ce que, pour la réalisation d'au moins une partie de l'élément de prothèse à partir de l'empreinte, le procédé comprend un relevé direct des contours de l'empreinte (14) pour en déterminer les cotes et, après transmission des données relevées au moyen d'enregistrement (21), une correction par programme des données relevées pour éliminer les antidépouilles, les données corrigées étant transmises aux moyens de façonnage susdits.

2. Procédé suivant la revendication 1, caractérisé en ce qu'on utilise un sachet d'allure conique, l'ouverture du sachet étant située à la base du cône.

3.  Procédé suivant l'une ou l'autre des revendications 1 et 2, caractérisé en ce qu'on utilise un sachet réalisé en un matériau élastique, tel que du polyéthylène.

4.  Procédé suivant l'une quelconque des revendications 1 à 3, caractérisé en ce que la matière (7) à utiliser pour la réalisation de l'empreinte est une matière à deux composants à base d'un élastomère de polysiloxane durcissable.

5.  Procédé suivant l'une quelconque des revendications 1 à 4, caractérisé en ce que le matériau utilisé pour le sachet n'adhère pas à la matière susdite, le sachet étant séparé de la matière durcie lorsque l'empreinte est dégagée de la cavité.

6.  Procédé suivant l'une quelconque des revendications 1 à 5, caractérisé en ce qu'on dispose, dans la cavité (2), une tige de centrage (9) pourvue, à son extrémité située dans la cavité, d'un bouchon 8, on injecte la matière dans ladite cavité et, pendant que cette matière est à l'état visqueux, on y enfonce une armature (38), qui est enfilée sur la tige de centrage (9), la section de cette armature décroissant régulièrement de l'ouverture de la cavité vers le fond de cette dernière.

7.  Procédé suivant la revendication 6, caractérisé en ce qu'on garnit l'armature (38) d'une reproduction de tête de prothèse (11), on place dans cette dernière un élément métallique (39) qui permet de constater, par radiographie, l'orientation de la reproduction de la tête de prothèse.

8.  Procédé suivant l'une quelconque des revendications 1 à 7, caractérisé en ce que, dans le cas d'une prothèse de la hanche, on réalise celle-ci en deux parties (33, 34), la première partie (33), façonnée à partir de l'empreinte de la cavité (21) réalisée dans le fémur, étant destinée à être logée dans ladite cavité, la seconde partie, présentant la tête de la prothèse (23), étant préfabriquée, on prévoit, dans l'axe de la première partie et à son extrémité (35) la plus volumineuse, un évidement tronconique (36) destiné à recevoir un prolongement (37), de forme correspondante, du col de la seconde partie, la rotation dudit prolongement dans l'évidement, autour de son axe, permettant de corriger l'antiversion et la rétroversion.

9.  Procédé suivant la revendication 8, caractérisé en ce que l'on prévoit dans la première partie de la prothèse des moyens permettant d'y fixer un organe de traction destiné à dégager cette première partie, en cas de nécessité, de la cavité précitée.

**Claims**

1.  Process for producing an implantable prosthesis element, made of hard material, for the total or partial replacement of a joint, in particular a hip joint, and intended to match the shapes of a cavity (2) made in the part of the skeleton (1) in question, which process comprises taking an impression (14) of this cavity and producing at least part of the prosthesis element from the said impression by transmitting data relating to the impression to a recording means, processing these recorded data and transmitting the processed data to shaping means capable of producing, in accordance with the transmitted data, the abovementioned prosthesis element from a blank made of a hard material suitable for a prosthesis, characterised in that, for taking an impression of the cavity, the process comprises lining the walls of the cavity (2) with a bag (3) made of a leakproof and elastic material, which is sterile and flexible and does not adhere to the said walls, so that its opening debouches to the outside of the cavity, filling the bag (3) with a sterile substance (7) which is capable of matching the walls of the cavity, is chosen from among substances not adhering to the material constituting the bag, hardens when cooled and has, in the hardened state, a modulus of elasticity such that the hardened substance can be extracted from the cavity without permanent deformation, and removing the substance enclosed in the bag (3) after its hardening in the cavity, and in that, for producing at least part of the prosthesis element from the impression, the process comprises direct plotting of the contours of the impression (14) in order to determine the details thereof and, after transmission of the observed data to the recording means (21), correction of the observed data by means of a program in order to eliminate the anti-reliefs, the corrected data being transmitted to the abovementioned shaping means.

2.  Process according to Claim 1, characterised in that a bag with a conical appearance is used, the opening of the bag being situated at the base of the cone.

3. Process according to either of Claims 1 and 2, characterised in that a bag is used which is made of an elastic material, such as polyethylene.

4. Process according to any one of Claims 1 to 3, characterised in that the substance (7) to be used for producing the impression is a substance of two components based on a hardenable polysiloxane elastomer.

5. Process according to any one of Claims 1 to 4, characterised in that the material used for the bag does not adhere to the abovementioned substance, the bag being separated from the hardened substance when the impression is removed from the cavity.

6. Process according to any one of Claims 1 to 5, characterised in that there is arranged, in the cavity (2), a centring rod (9) provided, at its end situated in the cavity, with a plug (8), the substance is injected into the said cavity and, while the substance is in the viscous state, a reinforcement (38) is driven in, which reinforcement is passed over the centring rod (9), the cross-section of this reinforcement decreasing uniformly from the opening of the cavity towards the bottom of the latter.

7. Process according to Claim 6, characterised in that the reinforcement (38) is fitted with a prosthesis head reproduction (11), a metallic element (39) is placed in the latter and makes it possible to determine, by radiography, the orientation of the prosthesis head reproduction.

8. Process according to any one of Claims 1 to 7, characterised in that, in the case of a hip prosthesis, the latter is made in two parts (33, 34), the first part (33), modelled from the impression of the cavity (21) formed in the femur, being intended to be lodged in the said cavity, the second part, presenting the prosthesis head (23), being prefabricated, and in that there is provided, in the axis of the first part and at its end (35) of greatest volume, a truncated recess (36) intended to receive an extension (37), of corresponding shape, of the neck of the second part, the rotation of the said extension in the recess, about its axis, making it possible to correct anteversion and retroversion.

9. Process according to Claim 8, characterised in that there are provided, in the first part of the prosthesis, means permitting the attachment of a traction member intended to release this first part, if required, from the abovementioned cavity.

**Ansprüche**

1. Verfahren zur Herstellung eines implantierbaren Prothesenelementes aus hartem Material, das vollständig oder teilweise gegen ein Gelenk, insbesondere ein Hüftgelenk, ausgetauscht wird und dazu vorgesehen ist, sich an einen im betreffenden Skeletteil (1) ausgebildeten Hohlraum (2) anzuschmiegen, wobei das Verfahren das Nehmen eines Abdruckes (14) von diesem Hohlraum und die Ausbildung wenigstens eines Teils des Prothesenelementes anhand dieses Abdruckes durch die Übertragung der den Abdruck betreffenden Daten an ein Aufzeichnungsmittel, die Verarbeitung dieser aufgezeichneten Daten und die Übertragung der verarbeiteten Daten an Formgebungsmittel, die gemäß diesen übertragenen Daten das oben genannte Prothesenelement aus einem Rohling aus einem für eine Prothese geeigneten, harten Material herstellen können, umfaßt, dadurch gekennzeichnet, daß das Verfahren zum Nehmen des Abdruckes vom Hohlraum eine Auskleidung der Wände des Hohlraums (2) mittels eines Sackes (3), der aus einem dichten und elastischen Material hergestellt ist, das steril und geschmeidig ist und nicht an den Wänden haftet, derart, daß er in das Äußere des Hohlraums geöffnet ist, eine Ausfüllung des Sackes (3) mit einer sterilen Substanz (7), die sich an die Wände des Hohlraums anschmiegen kann und die aus solchen Substanzen ausgewählt wird, die nicht am den Sack bildenden Material haftet, die kalthärtet und im gehärteten Zustand ein Elastizitätsmodul besitzt, derart, daß die gehärtete Substanz aus dem Hohlraum ohne bleibende Verformung herausgezogen werden kann, und ein Abheben der im Sack (3) eingeschlossenen Substanz, nachdem sie im Hohlraum gehärtet ist, umfaßt und daß das Verfahren für die Ausbildung wenigstens eines Teils des Prothesenelementes anhand des Abdruckes eine direkte Vermessung der Konturen des Abdruckes (14) umfaßt, um daraus die Maße und nach der Übertragung der Vermessungsdaten an das Aufzeichnungsmittel (21) eine programmgesteuerte Korrektur zur Beseitigung nicht ausgewerteter Bereiche festzulegen, wobei die korrigierten Daten an die oben genannten Formgebungsmittel übertragen werden.

2. Verfahren gemäß Anspruch 1, dadurch gekennzeichnet, daß ein Sack mit kegelförmigem Verlauf verwendet wird, wobei sich die Öffnung des Sackes an der Basis des Kegels befindet.

3. Verfahren gemäß dem einen oder dem anderen der Ansprüche 1 und 2, dadurch gekennzeichnet, daß ein Sack verwendet wird, der aus einem elastischen Material wie etwa aus Polyethylen hergestellt ist.

4. Verfahren gemäß einem der Ansprüche 1 bis 3, dadurch gekennzeichnet, daß die für die Herstellung des Abdruckes verwendete Substanz (7) eine Zweikomponentensubstanz auf der Basis eines härtbaren Polysiloxsan-Elastomers ist.

5. Verfahren gemäß einem der Ansprüche 1 bis 4, dadurch gekennzeichnet, daß das für den Sack verwendete Material nicht an der oben genannten Substanz haftet, wobei der Sack vom gehärteten Material abgenommen wird, wenn der Abdruck vom Hohlraum abgelöst ist.

6. Verfahren gemäß einem der Ansprüche 1 bis 5, dadurch gekennzeichnet, daß im Hohlraum (2) ein Zentrierstift (9) angeordnet wird, der an seinem im Hohlraum befindlichen Ende mit einer Abschlußkappe (8) versehen ist, daß die Substanz in den Hohlraum hineingedrückt wird und daß dann, wenn sich diese Substanz in einem zähflüssigen Zustand befindet, eine Ummantelung (38), die auf den Zentrierstift (9) aufgeschoben wird, in sie hineingetrieben wird, wobei der Querschnitt dieser Ummantelung gleichmäßig von der Öffnung des Hohlraums bis zum Boden des letzteren abnimmt.

7. Verfahren gemäß Anspruch 6, dadurch gekennzeichnet, daß die Ummantelung (38) mit einer Nachbildung des Prothesenkopfes bestückt wird, daß in diesen letzteren ein metallisches Element (39) eingesetzt wird, das mittels Radiographie die Orientierung der Nachbildung des Prothesenkopfes festzustellen erlaubt.

8. Verfahren gemäß einem der Ansprüche 1 bis 7, dadurch gekennzeichnet, daß im Falle einer Hüftprothese diese aus zwei Teilen (33, 34) hergestellt wird, wobei der erste Teil (33), der anhand des Abdruckes des im Oberschenkelknochen ausgebildeten Hohlraums (21) geformt wird, dazu vorgesehen ist, in diesen Hohlraum eingesetzt zu werden, wobei der den Prothesenkopf (23) bildende zweite Teil vorgefertigt ist, und daß auf der Mittellinie des ersten Teils und an seinem das größte Volumen besitzenden Ende (35) eine kegelstumpfartige Vertiefung (36) vorgesehen ist, die der Aufnahme einer entsprechend geformten Verlängerung (37) des Halses des zweiten Teils dient, wobei die Drehung der Verlängerung in der Aussparung um ihre Mittellinie die Korrektur der Antiversion und der Retroversion erlaubt.

9. Verfahren gemäß Anspruch 8, dadurch gekennzeichnet, daß im ersten Teil der Prothese Mittel vorgesehen sind, die dort ein Zugelement zu befestigen erlauben, das, falls dies erforderlich ist, der Ablösung dieses ersten Teils vom genannten Hohlraum dient.

FIG.1

FIG. 2

FIG. 3

FIG.4

FIG.7

FIG. 8

FIG. 9

FIG. 5

FIG. 6

FIG. 10